# EUROPEAN PATENT SPECIFICATION

(11) **EP 4 561 450 B1**
(45) Date of publication and mention of the grant of the patent: **30.07.2025**
(21) Application number: 23745395.6
(22) Date of filing: 26.06.2023
(51) Int. Cl.: A61B 6/04, A61B 6/00

(54) **MEDICAL IMAGING DEVICE**
MEDIZINISCHE BILDGEBUNGSVORRICHTUNG
DISPOSITIF D'IMAGERIE MÉDICALE

(30) Priority: 31.08.2022 CN 202222299280 U
(43) Date of publication of application: 04.06.2025
(73) Proprietor: Siemens Shanghai Medical Equipment Ltd., 201318 Shanghai (CN)
(72) Inventor: SHE, Wen Zhi, Shanghai 201318 (CN)
(74) Representative: HKW Intellectual Property PartG mbB
(86) International application number: PCT/CN2023/102457
(87) International publication number: WO 2024/045810

(56) References cited:
- US-A- 4 082 955
- US-A- 4 408 341
- US-A- 4 450 575

## Description

### TECHNICAL FIELD

The disclosure relates to the technical field of medical appliances, and in particular to a medical imaging device.

### BACKGROUND

In the medical field, medical imaging devices are often used to emit rays to examine patients radiologically and generate images. Medical staffs make medical diagnosis by observing images. In order to facilitate the medical staff to operate the X-ray tube assembly on the medical imaging device to examine patients, the X-ray tube assembly is mounted on a column, and a ground rail is provided on the ground near the medical imaging device. The column can slide along the ground rail so as to drive the X-ray tube assembly to slide. However, the alignment process of the medical imaging device is complex.

US 4,408,341 discloses an X-ray examination apparatus, comprising a frame, a patient examination table, slidably connected to the frame a guide rail extending in a longitudinal direction a column, movable on the guide rail in the longitudinal direction, and an X-ray source on the column. The X-ray examination apparatus further comprises a carriage, movable on the frame in the longitudinal direction, the guide rail being movable on the carriage in the longitudinal direction; and linkage means for assuring that when the carriage, the guide rail, or the column are moved relative to the frame, the guide rail is moved in the same direction as the carriage but at twice the distance relative to the frame, and the column is moved in the same direction as the carriage but at three times the distance relative to the frame.

US 4,450,575 and US 4,082,955 show, respectively, a further X-ray apparatus.

### SUMMARY OF THE INVENTION

An object of the invention is to provide a medical imaging device according to claim 1. Preferred embodiments are included as dependent claims.

### SUMMARY OF THE DISCLOSURE

An object of the disclosure is to provide a medical imaging device, which has the advantages of simple alignment process and high mounting efficiency.

The disclosure provides a medical imaging device, including: a sliding seat, a bracket, a column and an X-ray tube assembly. The sliding seat is fixedly arranged on a bed of the medical imaging device, and the sliding seat has a slideway extending along a length direction of the bed. The bracket is provided with a moving component and a restraining member. The moving component moves along the slideway, and the restraining member is fitted with the sliding seat to limit a displacement of the bracket in a width direction of the bed. The column is vertically arranged on the bracket. The X-ray tube assembly is arranged on the column. The sliding seat includes a rail seat body. The rail seat body is provided with a bottom edge, a connecting edge, a top edge and a bend edge that are connected sequentially. The bottom edge and the top edge are arranged opposite to each other. The connecting edge and the bend edge are opposite to each other and both perpendicular to the bottom edge. The bottom edge, the connecting edge, the top edge and the bend edge together form the slideway, and the moving component is located in the slideway and contacts the bottom edge. The medical imaging device further includes a first auxiliary support member. The first auxiliary support member is located in the slideway. Along a height direction of the bed, a distance between the first auxiliary support member and a bottom surface of the slideway is a first distance, and a distance between the first auxiliary support member and a top surface of the slideway is a second distance. The second distance is configured such that when the X-ray tube assembly rotates around a central axis of the column to make the column have a tendency to overturn, the first auxiliary support member is capable of abutting against the top edge.

According to the medical imaging device provided by embodiments of the disclosure, the sliding seat is fixedly arranged on the bed. The sliding seat has the slideway extending along the length direction of the bed. The bracket for the arrangement of the column is provided with the moving component. The moving component moves along the slideway, that is, the sliding seat that provides the slideway for the column to slide is mounted on the bed. In this way, the sliding seat and the bed are integrated into a whole. Therefore, during the alignment of the medical imaging device, it is only required to make the bed horizontal, which simplifies the alignment process and thereby is beneficial to reduce the mounting time and improve the mounting efficiency.

In some embodiments, along a height direction of the bed, there is a gap between the moving component and a top surface of the slideway.

In some embodiments, the moving component includes two first support bearings. The two first support bearings are spaced apart along the length direction of the bed.

In some embodiments, a dodging opening is formed between the bend edge and the bottom edge. The moving component is connected to the bracket through a connecting member. The dodging opening is configured to allow the connecting member to pass through, and a size of the moving component along a height direction of the bed is greater than a size of the dodging opening along the height direction of the bed.

In some embodiments, the first auxiliary support member includes two first rolling bearings. The two first rolling bearings are spaced apart along the length direction of the bed, and the first rolling bearings are connected to the bracket through a first eccentric shaft such that the second distance is adjustable.

In some embodiments, the sliding seat further includes a cylinder. The cylinder extends along the length direction of the bed. The restraining member includes a pair of first abutting members and a pair of second abutting members. The first abutting members are located on a front side of the cylinder away from the column and abut against the cylinder, or the first abutting members are located on a front side of the bend edge away from the column and abut against the bend edge. The second abutting members are located on a rear side of the cylinder near the column and abut against the cylinder, or the second abutting members are located on a rear side of the bend edge near the column and abut against the bend edge.

In some embodiments, the cylinder is located above the rail seat body, the first abutting members abut against the cylinder, and the second abutting members abut against the bend edge. The medical imaging device further includes a second auxiliary support member and a third auxiliary support member. The second auxiliary support member is located on the rear side of the cylinder, and along the width direction of the bed, a distance between the second auxiliary support member and the cylinder is a third distance. The third auxiliary support member is located on the front side of the bend edge and in the slideway, and along the width direction of the bed, a distance between the third auxiliary support member and the bend edge is a fourth distance.

The third distance and the fourth distance are configured such that when the X-ray tube assembly receives an acting force to make the column have a tendency to overturn, the second auxiliary support member is capable of abutting against the cylinder and the third auxiliary support member is capable of abutting against the bend edge.

In some embodiments, the second auxiliary support member includes two second rolling bearings. The two second rolling bearings are spaced apart along the length direction of the bed, and the second rolling bearings are connected to the bracket through a second eccentric shaft such that the third distance is adjustable. The third auxiliary support member includes two third rolling bearings. The two third rolling bearings are spaced apart along the length direction of the bed, and the third rolling bearings are connected to the bracket through a third eccentric shaft such that the fourth distance is adjustable.

In some embodiments, the bracket includes a support plate, and a first mounting plate and a second mounting plate that are perpendicularly connected to the support plate. The first abutting members and the second auxiliary support member are fixed to the first mounting plate, and the second abutting members and the third auxiliary support member are fixed to the second mounting plate.

In some embodiments, the medical imaging device further includes a fourth auxiliary support member fixedly arranged on the bracket. The fourth auxiliary support member is located on the rear side of the bend edge and abuts against the bend edge, or the fourth auxiliary support member is located on the rear side of the cylinder and abuts against the cylinder.

In some embodiments, the sliding seat further includes a supporting table fixedly arranged on the bed. The cylinder and the rail seat body are both fastened and connected to the supporting table. One of the cylinder and the rail seat body is connected to a top surface of the supporting table, and the other is connected to a bottom surface of the supporting table.

### BRIEF DESCRIPTION OF THE DRAWINGS

The embodiments of the disclosure will be described in detail below with reference to the accompanying drawings, so that the above and other features and advantages of the disclosure will be made clearer to those of ordinary skill in the art. In the figures:
FIG. 1 is a schematic front view of a medical imaging device in the related art;
FIG. 2 is a schematic top view of the medical imaging device in the related art;
FIG. 3 is a schematic structure diagram of a medical imaging device when an X-ray tube assembly is in a working position according to an embodiment of the disclosure;
FIG. 4 is a schematic diagram of the medical imaging device shown in FIG. 3 with part of the structure omitted;
FIG. 5 is a schematic diagram of the medical imaging device shown in FIG. 3 when a sliding seat is fitted with a bracket;
FIG. 6 is a schematic structure diagram of the bracket in FIG. 5;
FIG. 7 is a schematic diagram of a medical imaging device of another embodiment when a sliding seat is fitted with a bracket;
FIG. 8 is a schematic diagram of a medical imaging device of yet another embodiment when a sliding seat is fitted with a bracket;
FIG. 9 is a schematic diagram of FIG. 5 when the sliding seat is fitted with the bracket;
FIG. 10 is a partial schematic diagram of FIG. 9 when a rail seat body is fitted with the bracket;
FIG. 11 is a schematic cross-sectional view of FIG. 5 when the sliding seat is fitted with the bracket;
FIG. 12 is a partial schematic diagram I of the bracket shown in FIG. 6; and
FIG. 13 is a partial schematic diagram II of the bracket shown in FIG. 6.

### Reference signs:

100-medical imaging device;
110-bed;
120-sliding seat; 121-slideway; 122-rail seat body; 1221-bottom edge; 1222-connecting edge; 1223-top edge; 1224-bend edge; 1225-first edge; 1226-second edge; 1227-third edge; 123-guide rail bar; 124-cylinder; 125-supporting table; 126-housing;
130-bracket; 131-support plate; 132-first mounting plate; 133-second mounting plate;
140-moving component; 141-first support bearing; 142-sliding block; 143-connecting member;
150-first auxiliary support member; 151-first eccentric shaft;
160-restraining member; 161-first abutting member; 162-second abutting member; 163-second auxiliary support member; 164-third auxiliary support member; 165-fourth auxiliary support member; 1651-deep groove ball bearing; 1652-fixed plate;
170-column;
180-X-ray tube assembly.

### DETAILED DESCRIPTION

In order to have a clearer understanding of the technical features, objects and effects of the disclosure, specific implementations of the disclosure will be described with reference to the accompanying drawings, in which the same reference numerals denote the same parts.

Here, "schematic" means "serving as a case, example, or illustration", and any illustration or implementation described herein as "schematic" should not be interpreted as a more preferred or more advantageous technical solution.

For the sake of brevity, only the parts related to the disclosure are schematically shown in the drawings, which do not represent the actual structure of the product. In addition, in order to make the drawings simple and easy to understand, for the components having the same structure or function in some drawings, only one of them is schematically illustrated, or only one of them is marked.

"One" herein not only means "only one", but also means "more than one". "First" and "second" are only used to distinguish each other, not to indicate their importance and sequence, or the premise of their mutual existence, etc.

It should be noted that the directional indications (such as on, under, left, right, front, rear, ...) in the embodiments of the disclosure are used merely to explain the relative position relationship, movement, etc. between the components in a particular attitude (as shown in the accompanying drawings), and if the particular attitude changes, the directional indications change accordingly.

In the disclosure, unless otherwise explicitly specified and defined, the terms "connection", "fixation" and the like shall be understood broadly. For example, the "fixation" may be a fixed connection, a detachable connection or integration; or may be a mechanical connection or an electrical connection; or may be a direct connection, an indirect connection through an intermediate medium, or an internal communication between two components or interaction between two components, unless otherwise specified. For those of ordinary skill in the art, the specific meaning of the above terms in the disclosure can be understood according to specific situations.

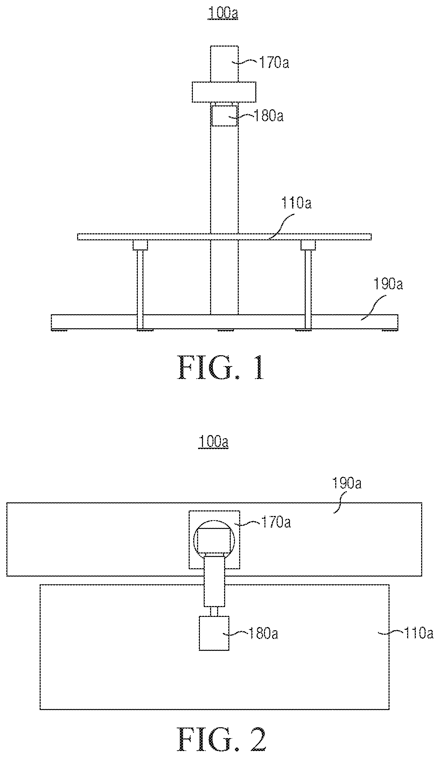

FIG. 1 is a schematic front view of a medical imaging device 100a in the related art. FIG. 2 is a schematic top view of the medical imaging device 100a in the related art. Referring to FIG. 1 and FIG. 2, in an example of the related art, the medical imaging device 100a includes a bed 110a, a column 170a, a ground rail 190a and an X-ray tube assembly 180a. The ground rail 190a is arranged on the ground near the bed 110a and extends along a length direction of the bed 110a. The column 170a is slidably arranged on the ground rail 190a. The X-ray tube assembly 180a is arranged on the column 170a. In this way, the column 170a can slide along the ground rail 190a so as to drive the X-ray tube assembly 180a to slide.

However, the inventors found that after the medical imaging device 100a is assembled, during the adjustment, it is required not only to make bed 110a and the ground rail 190a horizontal separately, but also to calibrate the accuracy between the bed 110a and the ground rail 190a. This leads to complex adjustment process, long mounting time and low mounting efficiency of the medical imaging device 100a. In addition, in order to ensure high stability of the medical imaging device 100a, the medical imaging device is further provided with a support structure for supporting the ground rail 190a and the column 170a, which leads to complex structure and high manufacturing cost of the medical imaging device 100a. After careful study, the inventors found that the main reason for the above problems of the medical imaging device 100a is that the ground rail 190a and the bed 110a are separated, so they need to be supported and aligned separately.

In view of this, the inventors have come up with a medical imaging device. In this medical imaging device, the column is mounted on the bracket, the bracket is provided with a moving component, and a sliding seat fitted with the moving component is fixedly arranged on the bed, rather than being independent of the bed. Further, the inventors also have found that the medical imaging device is unstable in use. Therefore, the inventors design the medical imaging device to further have a restraining member, so as to ensure that the bracket can move only along the length direction of the bed. The medical imaging device in the disclosure can be various X-ray medical imaging devices, especially X-ray medical imaging devices containing a bed.

The implementation manners of the medical imaging device provided by embodiments of the disclosure will be described in detail below in conjunction with accompanying drawings.

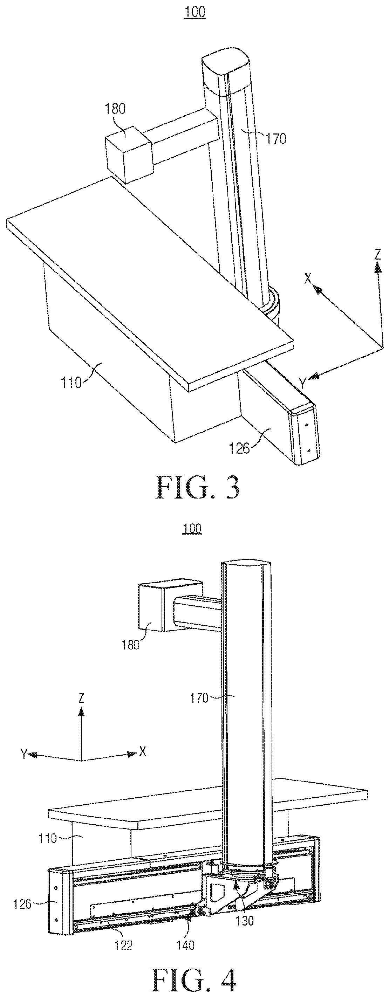

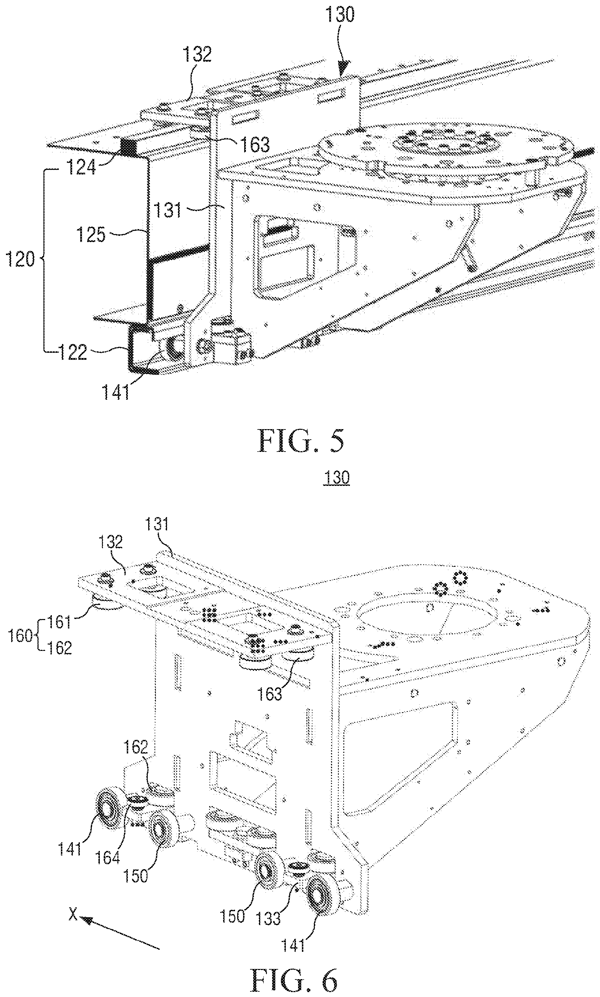

FIG. 3 is a schematic structure diagram of a medical imaging device 100 when an X-ray tube assembly 180 is in a working position according to an embodiment of the disclosure. FIG. 4 is a schematic diagram of the medical imaging device 100 shown in FIG. 3 with part of the structure omitted. FIG. 5 is a schematic diagram of the medical imaging device 100 shown in FIG. 3 when a sliding seat 120 is fitted with a bracket. Referring to FIG. 3 to FIG. 5, the medical imaging device 100 includes a bed 110, a sliding seat 120, a bracket 130, a column 170 and an X-ray tube assembly 180. The bed 110 is fixed to the ground. The bed 110 is available for the patient to lie flat. The sliding seat 120 is fixedly arranged on the bed 110. The bracket 130 is connected to the sliding seat 120. The column 170 is vertically arranged on the bracket 130. The X-ray tube assembly 180 is arranged on the column 170.

The bracket 130 can slide along a length direction (X direction in FIG. 3) of the bed 110 relative to the sliding seat 120, so that the column 170 and the X-ray tube assembly 180 can move along the X direction. The column 170 can rotate around its central axis relative to the bracket 130, so that the X-ray tube assembly 180 can rotate around the central axis of the column 170. The X-ray tube assembly 180 can slide up and down along a height direction (Z direction in FIG. 3) of the bed 110 relative to the column 170. In this way, medical staff can conveniently move the X-ray tube assembly 180 to a proper position so as to examine the patient. It should be understood that the X-ray tube assembly 180 has a working position and an idle position. In FIG. 3, when the X-ray tube assembly 180 is in the working position, an orthographic projection of the X-ray tube assembly in the Z direction may fall on the bed 110. When the patient lies on the bed 110, X-rays emitted downward from the X-ray tube assembly 180 can be projected onto the patient's body, thereby examining the patient.

The bracket 130 can slide along the X direction relative to the sliding seat 120 in the following specific implementations:
In a first example, referring to FIG. 4 to FIG. 6, the sliding seat 120 is arranged on one side of the bed 110 along its width direction (Y direction in FIG. 4). The sliding seat 120 includes a rail seat body 122. The rail seat body 122 is configured to have a groove extending along the X direction, and the groove forms a slideway. The bracket 130 is provided with a first support bearing 141. The first support bearing 141 moves along the slideway on the rail seat body 122. The bracket 130 slides along the slideway along with the first support bearing 141. FIG. 6 is a schematic structure diagram of the bracket 130 in FIG. 5.

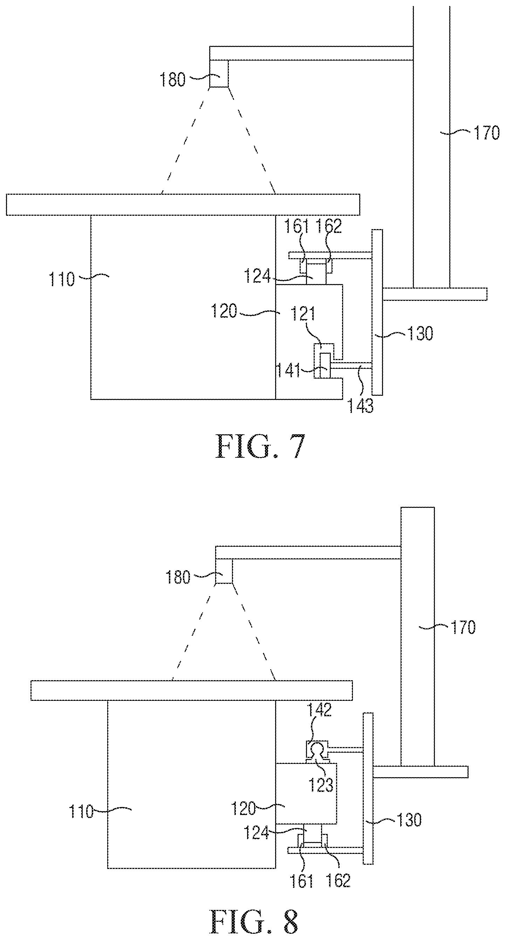

In a second example, referring to FIG. 7, the sliding seat 120 may be a blocky structure. The sliding seat 120 is provided with a groove extending along the X direction, and the groove forms a slideway 121. The bracket 130 is provided with a first support bearing 141. The first support bearing 141 contacts the groove and slides along the slideway 121. The bracket 130 slides along the slideway 121 along with the first support bearing 141. FIG. 7 is a schematic diagram of a medical imaging device 100 of another embodiment when the sliding seat 120 is fitted with the bracket 130.

It should be noted that in the above two examples, the first support bearing 141 may be a sliding bearing, and in this case, the first support bearing 141 slides in the slideway 121 so as to drive the bracket 130 to move along the X direction. Alternatively, the first support bearing 141 may also be a rolling bearing, and in this case, the first support bearing 141 rolls in the slideway 121 so as to drive the bracket 130 to move along the X direction. In the above two examples, the first support bearing 141 may also be replaced with a roller wheel, and the roller wheel rolls in the slideway 121 so as to drive the bracket 130 to move along the X direction. The number of the first support bearings 141 may be two.

Based on the above, the medical imaging device 100 of this embodiment has the sliding seat 120 and the bracket 130. The sliding seat 120 is provided with the slideway 121 extending along the X direction (for example, the groove formed in the rail seat body 122, or the groove formed in the sliding seat 120 in FIG. 7). The bracket 130 is provided with a moving component 140. The moving component 140 may include the two first support bearings 141 or the two roller wheels spaced apart along the X direction. In the implementation where the moving component 140 includes the two first support bearings 141, the two first support bearings 141 can move in the slideway 121 in a sliding or rolling manner, thereby driving the bracket 130 and the column 170 to move. In the implementation where the moving component 140 includes the two roller wheels, the two roller wheels roll in the slideway 121 so as to drive the bracket 130 and the column 170 to move along the slideway 121.

It should be understood that since the number of the first support bearings 141 is two, the two first support bearing 141 each have a contact point with the slideway 121, the two contact points can form a straight line, and the contact reference of the moving component 140 with the slideway 121 is line contact. Therefore, the straightness of the motion path of the moving component 140 can be ensured, so that the column 170 can smoothly slide along a straight line, thereby reducing the probability of the moving component 140 getting stuck, which may cause unsmooth sliding of the X-ray tube assembly 180 and bring bad experience to the patient.

In a third example, referring to FIG. 8, the sliding seat 120 may be a blocky structure. The sliding seat 120 is provided with a guide rail bar 123 extending along the X direction. The slideway 121 is formed on the guide rail bar 123. The moving component 140 may be a sliding block 142. The sliding block 142 is slidably mounted on the guide rail bar 123. The sliding block 142 slides along the guide rail bar 123 so as to drive the bracket 130 to move along the X direction. FIG. 8 is a schematic diagram of a medical imaging device 100 of yet another embodiment when the sliding seat 120 is fitted with the bracket 130. It can be understood that in some embodiments, the sliding block 142 may be replaced with a slide rail fitted with the guide rail bar 123, which is not limited in this embodiment. Specifically, the guide rail bar 123 is commercially available, and thus, is easy to obtain.

It should be understood that the moving component 140 contacts the slideway 121, and the sliding seat 120 also supports the moving component 140 so as to support the bracket 130, the column 170 and the X-ray tube assembly 180. The following description is an example in which the sliding seat 120 includes the rail seat body 122, the slideway 121 is the groove formed in the rail seat body 122 and the moving component 140 includes the first support bearings 141.

In addition, referring to FIG. 4 to FIG. 8, the bracket 130 is further provided with a restraining member 160, and the restraining member 160 is fitted with the sliding seat 120 to limit a displacement of the bracket 130 in the Y direction. This can ensure the bracket 130 to move only along the X direction as much as possible, which helps in reducing the possibility of overturning or shaking of the column 170 due to the movement of the bracket 130 along the Y direction, thereby improving the stability of the column 170. In this way, the X-rays emitted from the X-ray tube assembly 180 in the working position can be stably projected onto the bed 110 as much as possible, so that high-quality images can be obtained.

Based on the above, according to the medical imaging device 100 of this embodiment, the sliding seat 120 is fixedly arranged on the bed 110, the sliding seat 120 has the slideway 121 extending along the X direction, the bracket 130 for the arrangement of the column 170 is provided with the moving component 140, and the moving component 140 moves along the slideway 121. Thus, in the medical imaging device 100, the sliding seat 120 that provides the slideway 121 for the column 170 to slide is mounted on the bed 110. In this way, the sliding seat 120 and the bed 110 are integrated into a whole. Therefore, during the alignment of the medical imaging device 100, it is only required to make the bed 110 horizontal, which simplifies the alignment process and thereby is beneficial to reduce the mounting time and improve the mounting efficiency. Moreover, there is no need to provide an additional support structure to support the sliding seat 120, which avoids increasing the components, thereby avoiding complicating the structure of the medical imaging device 100 and helping in avoiding an increase in the manufacturing cost.

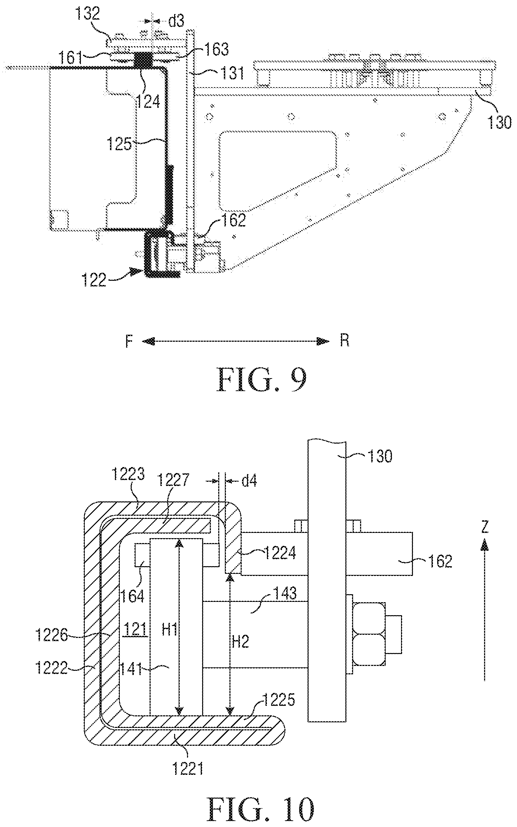

FIG. 9 is a schematic diagram of FIG. 5 when the sliding seat 120 is fitted with the bracket 130. FIG. 10 is a partial schematic diagram of FIG. 9 when the rail seat body 122 is fitted with the bracket 130. Referring to FIG. 9 and FIG. 10, when the sliding seat 120 includes the rail seat body 122, the rail seat body 122 is provided with a bottom edge 1221, a connecting edge 1222, a top edge 1223 and a bend edge 1224 that are connected sequentially. The bottom edge 1221 and the top edge 1223 are arranged opposite to each other. The connecting edge 1222 and the bend edge 1224 are opposite to each other and both perpendicular to the bottom edge 1221. The connecting edge 1222 is located on the same side of the bottom edge 1221 and the top edge 1223. The bottom edge 1221, the connecting edge 1222, the top edge 1223 and the bend edge 1224 together form the slideway 121.

Further, the rail seat body 122 may be further provided with a first edge 1225, a second edge 1226 and a third edge 1227 connected sequentially. An end of the first edge 1225 away from the second edge 1226 is connected to an end of the bottom edge 1221 away from the connecting edge 1222. The first edge 1225 is closely attached to the bottom edge 1221, the second edge 1226 is closely attached to the connecting edge 1222, and the third edge 1227 is closely attached to the top edge 1223. This can increase the structural strength of the rail seat body 122, thereby improving the stability of the first support bearing 141 and the bracket 130. In this embodiment, all the edges constituting the rail seat body 122 together form the slideway 121. It should be understood that the rail seat body 122 may be a sheet metal structure, which is made by bending many times. Exemplarily, a flat plate may be folded once first, so that the first part and the second part of the flat plate are stacked along the thickness direction of the flat plane and the third part of the flat plate protrudes; the third part is bent to obtain the bend edge 1224; and the first part and the second part are bent twice.

In an example shown in FIG. 10, an end of the bend edge 1224 away from the top edge 1223 does not contact the bottom edge 1221, so that a dodging opening is formed between the bend edge 1224 and the bottom edge 1221. The dodging opening can dodge the connecting member 143, so that the connecting member 143 can pass through the dodging opening so as to connect the first support bearing 141 with the bracket 130. Moreover, a size H1 of the first support bearing 141 along the Z direction is greater than a size H2 of the dodging opening along the Z direction, so that the first support bearing 141 cannot pass through the dodging opening, which prevents the first support bearing 141 from coming out of the dodging opening. Thus, the first support bearing 141 can remain fitted with the slideway 121, and the sliding seat 120 can support the bracket 130, thereby ensuring the sustained use of the medical imaging device 100.

As shown in FIG. 10, along the Z direction, there is a gap between the first support bearing 141 and a top surface of the slideway 121. In this way, the first support bearing 141 only contacts a bottom surface of the slideway 121 and does not contact the top surface of the slideway 121, thereby reducing the possibility of the first support bearing 141 getting stuck due to the contact of the first support bearing 141 with both the bottom surface and the top surface of the slideway 121. Thus, the bracket 130 and the column 170 can smoothly slide along the slideway 121.

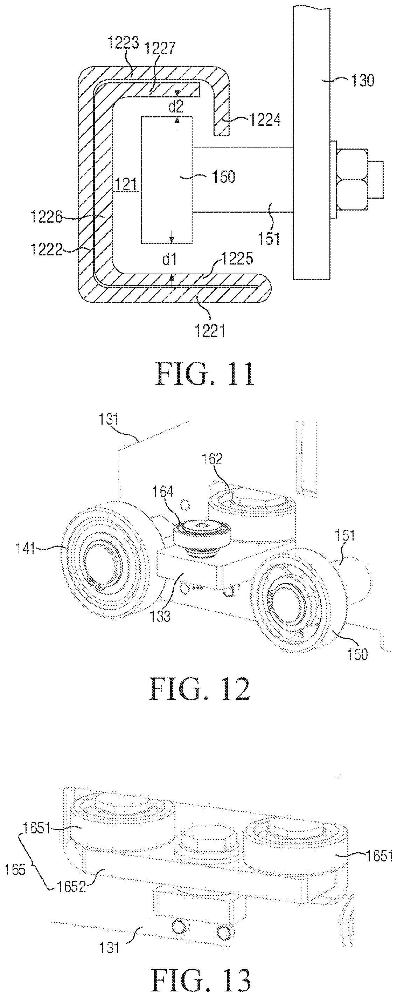

FIG. 11 is a schematic cross-sectional view of FIG. 5 when the sliding seat 120 is fitted with the bracket 130. Referring to FIG. 6 and FIG. 11, a groove is formed in the sliding seat 120. When the groove is formed as the slideway 121, the bracket 130 is further provided with a first auxiliary support member 150. The first auxiliary support member 150 is located in the slideway 121. When the X-ray tube assembly 180 is in the working position, the first auxiliary support member 150 has a first distance d1 (d1>0 mm) from the bottom surface of the slideway 121 in the Z direction. The first auxiliary support member 150 has a second distance d2 (d2>0 mm) from the top surface of the slideway 121 in the Z direction. The first distance d1 and the second distance d2 may be designed to be the same or different, for example, d2<d1. When the X-ray tube assembly 180 is in the working position, its center of gravity is directly in front of the central axis of the column 170, and the first auxiliary support member 150 contacts neither the top surface of the slideway 121 nor the bottom surface of the slideway 121, that is, the first auxiliary support member 150 does not function as a support. At this time, the column 170 may be regarded as being in a balanced state. Here, the front side refers to a side of the column 170 facing the bed 110 along the Y direction (denoted by arrow F in FIG. 9). Accordingly, the rear side refers to a side of the column 170 facing away from the bed 110 along the Y direction (denoted by arrow R in FIG. 9), and the left side and the right side are two sides of the column 170 along the X direction.

The second distance d2 is further configured such that when the X-ray tube assembly 180 rotates around the central axis of the column 170 to make the column 170 have a tendency to overturn, the first auxiliary support member 150 is capable of abutting against the top surface of the slideway 121. It should be understood that after the medical staff finishes examining the patient, they may operate the X-ray tube assembly 180 in the working position to rotate around the central axis of the column 170 along the counterclockwise or clockwise direction, for example, by 90°, so that the X-ray tube assembly 180 moves away from the bed 110 to the idle position, which prevents a stand-up patient from hitting the X-ray tube assembly 180. When the X-ray tube assembly 180 is rotated by 90° from the working position, the center of gravity of the X-ray tube assembly 180 deviates to the left side or the right side of the column 170, and the column 170 loses its balance, so that the column 170 and the bracket 130 connected to the column 170 tilt to the left side or the right side correspondingly. With this design of this embodiment, when the column 170 and the bracket 130 tilt to the left side or the right side, the bracket 130 drives the first support bearing 141 and the first auxiliary support member 150 to tilt, the first support bearing 141 is out of contact with the bottom surface of the slideway 121, and the first auxiliary support member 150 abuts against the top surface of the slideway 121. Since the sliding seat 120 is fixed to the bed 110, due to the limit of the sliding seat 120, the first auxiliary support member 150, the bracket 130 and the column 170 cannot tilt further.

Thus, when the X-ray tube assembly 180 is in the working position, the first auxiliary support member 150 contacts neither the top surface nor the bottom surface of the slideway 121, thereby preventing the first support bearing 141 from getting stuck. Moreover, the first auxiliary support member 150 can avoid the risk of the column 170 overturning due to the change of the center of gravity of the X-ray tube assembly 180 after its position changes, so that the column 170 and the X-ray tube assembly 180 have high stability.

In this embodiment, the second distance d2 can be designed reasonably according to the model and size of the column 170 and the X-ray tube assembly 180 in the medical imaging device 100. For example, when the X-ray tube assembly 180 has a large weight, even if it rotates by a small angle, the column 170 will have a tendency to overturn. In this case, the second distance d2 should be smaller. Therefore, the second distance d2 can be small, so that when the column 170 tilts slightly after the center of gravity of the X-ray tube assembly 180 changes due to its position change, the first auxiliary support member 150 can abut against the top surface of the slideway 121, thereby preventing the column 170 from tilting seriously.

According to the example shown in FIG. 6 and FIG. 11, the first auxiliary support member 150 may specifically include two first rolling bearings. The two first rolling bearings are spaced apart along the X direction. An inner ring of the first rolling bearing is sleeved on one end of a first eccentric shaft 151, and the other end of the first eccentric shaft 151 is connected to the bracket 130 through a screw member. In this way, after the screw member is loosened, the second distance d2 can be adjusted by screwing the first eccentric shaft 151. Thus, the second distance d2 can be flexibly designed to better adapt to the actual working conditions. For example, when the X-ray tube assembly 180 has a large weight, the second distance d2 can be decreased. When the X-ray tube assembly 180 has a small weight, the second distance d2 can be increased. Of course, the first rolling bearings may also be replaced with other types of bearing structures or blocky structures.

The fit between the restraining member 160 and the sliding seat 120 will be described in detail below. The restraining member 160 may include a first abutting member 161 and a second abutting member 162. Along the Y direction, the first abutting member 161 and the second abutting member 162 are spaced apart.

For example, the first abutting member 161 may be located on a front side of the bend edge 1224 and abut against the bend edge 1224, and the second abutting member 162 may be located on a rear side of the bend edge 1224 and abut against the bend edge 1224. That is, the restraining member 160 abuts against the front side and the rear side of the bend edge 1224 at the same time, so that the bend edge 1224 restrains a displacement of the restraining member 160 in the Y direction.

For another example, as shown in FIG. 8, the sliding seat 120 may be further provided with a cylinder 124. The cylinder 124 extends along the X direction. The first abutting member 161 may be located on a front side of the cylinder 124 and abut against the cylinder 124, and the second abutting member 162 may be located on a rear side of the cylinder 124 and abut against the cylinder 124. That is, the restraining member 160 abuts against the front side and the rear side of the cylinder 124 at the same time, so that the cylinder 124 restrains a displacement of the restraining member 160 in the Y direction.

For still another example, as shown in FIG. 9, the sliding seat 120 may be further provided with a cylinder 124. The cylinder 124 extends along the X direction. The first abutting member 161 may be located on a front side of the cylinder 124 and abut against the cylinder 124, and the second abutting member 162 may be located on a rear side of the bend edge 1224 and abut against the bend edge 1224. In this case, the cylinder 124 prevents the restraining member 160 from moving to the rear side, and the bend edge 1224 prevents the restraining member 160 from moving to the front side. Alternatively, in other embodiments, the first abutting member 161 may be located on a front side of the bend edge 1224 and abut against the bend edge 1224, and the second abutting member 162 may be located on a rear side of the cylinder 124 and abut against the cylinder 124. In this case, the bend edge 1224 prevents the restraining member 160 from moving to the rear side, and the cylinder 124 prevents the restraining member 160 from moving to the front side.

With such design, the restraining member 160 can be fitted with the sliding seat 120 in many ways. Moreover, on the basis of the restraining member 160 abutting against the sliding seat 120, the sliding seat 120 can also support the bracket 130 along the Y direction. Here a cross-sectional shape of the cylinder 124 is not limited to the rectangle shown in FIG. 9, and may also be a circle or other shapes.

The number of the first abutting members 161 may be two. Thus, the first abutting members 161 have two contact points with the bend edge 1224 or the cylinder 124, the two contact points can form a straight line, and the contact reference of the first abutting members 161 with the bend edge 1224 or the cylinder 124 is line contact. Therefore, the first abutting members 161 have a high contact accuracy with the bend edge 1224 or the cylinder 124, and the first abutting members 161 are in good contact with the bend edge 1224 or the cylinder 124. Similarly, the number of the second abutting members 162 may also be two. Then, the second abutting members 162 have a high contact accuracy with the bend edge 1224 or the cylinder 124, and are in good contact with the bend edge 1224 or the cylinder 124.

Still referring to FIG. 9 and FIG. 10, in the implementation where the cylinder 124 is located above the rail seat body 122, the first abutting member 161 is located on the front side of the cylinder 124 and abuts against the cylinder 124 and the second abutting member 162 is located on the rear side of the bend edge 1224 and abuts against the bend edge 1224, the medical imaging device 100 may further include a second auxiliary support member 163 and a third auxiliary support member 164. The second auxiliary support member 163 is located on the rear side of the cylinder 124, a distance between the second auxiliary support member 163 and the cylinder 124 along the Y direction is a third distance d3. The third auxiliary support member 164 is located on the front side of the bend edge 1224, the third auxiliary support member 164 is located in the slideway 121, and a distance between the third auxiliary support member 164 and the bend edge 1224 along the Y direction is a fourth distance d4.

The third distance d3 and the fourth distance d4 are configured such that when an acting force is applied to the X-ray tube assembly 180 to make the column 170 have a tendency to overturn, the second auxiliary support member 163 is capable of abutting against the cylinder 124 and the third auxiliary support member 164 is capable of abutting against the bend edge 1224.

It should be understood that after the patient lies on the bed 110, when the medical staff examines the patient, they may apply a downward acting force on the X-ray tube assembly 180 in the working position, so that the X-ray tube assembly 180 slides downward along the column 170 to be close to the patient. Due to the large height of the column 170, when a large acting force is applied to the X-ray tube assembly 180 suspended on the top of the column 170, the column 170 may easily lose its balance and tilt to the front side. With this design of this embodiment, when the column 170 tilts to the front side, the bracket 130 tilts forward along with the column 170, the first abutting member 161 is separated from the cylinder 124, and the second abutting member 162 is separated from the bend edge 1224. The second auxiliary support member 163 abuts against the cylinder 124 forward, and the third auxiliary support member 164 abuts against the bend edge 1224 backward. Therefore, due to the limit of the cylinder 124 to the second auxiliary support member 163 and the limit of the bend edge 1224 to the third auxiliary support member 164, the column 170 and the bracket 130 cannot tilt forward further.

In this way, when the medical staff operate the X-ray tube assembly 180 in the working position to move down, the second auxiliary support member 163 and the third support member can prevent the column 170 from tilting forward, so that the column 170 and the X-ray tube assembly 180 have high stability in use.

Of course, when the medical imaging device 100 is in actual use, after finishing examining the patient, the medical staff may also apply an upward acting force to the X-ray tube assembly 180, so that the X-ray tube assembly 180 moves upward.

In this embodiment, the third distance d3 and the fourth distance d4 can be designed reasonably according to the model and size of the column 170 and the X-ray tube assembly 180 in the medical imaging device 100. For example, when the column 170 is long and thin, the X-ray tube assembly 180 receives a smaller acting force, and at this time, the column 170 has a tendency to overturn. In this case, the third distance d3 and the fourth distance d4 should be smaller.

Both the third distance d3 and the fourth distance d4 are smaller, and are further configured such that when the column 170 tilts forward slightly, the second auxiliary support member 163 is capable of abutting against the cylinder 124, and the third auxiliary support member 164 is capable of abutting against the bend edge 1224, thereby preventing the column 170 from tilting seriously. The third distance d3 and the fourth distance d4 may be the same or different. When the third distance d3 and the fourth distance d4 are different, the third distance d3 may be designed to be less than the fourth distance d4. This is because the third auxiliary support member 164 is located in the slideway 121. By designing the fourth distance d4 to be slightly greater than the third distance d3, when the fourth distance d4 is adjustable, the fourth distance d4 can be adjusted more conveniently.

Further, according to the example shown in FIG. 6, the bracket 130 may specifically include a support plate 131, a first mounting plate 132 and a second mounting plate 133. The first mounting plate 132 and the second mounting plate 133 are perpendicularly connected to the support plate 131. The first mounting plate 132 and the second mounting plate 133 are opposite to each other and are respectively located on an upper part and a lower part of the support plate 131. The first abutting member 161 and the second auxiliary support member 163 are fixed to the first mounting plate 132, and the second abutting member 162 and the third auxiliary support member 164 are fixed to the second mounting plate 133.

Exemplarily, the second auxiliary support member 163 may include two second rolling bearings. The two second rolling bearings are spaced apart along the X direction. The second rolling bearings are connected to the first mounting plate 132 through a second eccentric shaft, and the second eccentric shaft is perpendicular to the first mounting plate 132 along the Z direction. In this case, the third distance d3 can be adjusted by screwing the second eccentric shaft. Thus, the third distance d3 can be flexibly adjusted according to the actual working conditions. The number of the second rolling bearings is not limited to two, and may also be three or more. In the case where the number of the second rolling bearings is two, when the X-ray tube assembly 180 receives a downward force that makes the column 170 tilt forward, the two second rolling bearings abut against the cylinder 124 at the same time, so that the contact reference of the second rolling bearings with the cylinder 124 is line contact. Of course, the second rolling bearings may also be replaced with other types of bearing structures or blocky structures as long as they are capable of abutting against the cylinder 124.

The third auxiliary support member 164 may include two third rolling bearings. The two third rolling bearings are spaced apart along the X direction. The third rolling bearings are connected to the second mounting plate 133 through a third eccentric shaft. The third eccentric shaft is perpendicular to the second mounting plate 133 along the Z direction. Thus, the fourth distance d4 can be adjusted by screwing the third eccentric shaft, so that the fourth distance d4 can be flexibly designed to adapt to the actual working conditions. The number of the third rolling bearings is not limited to two, and may also be three or more. In the case where the number of the third rolling bearings is two, when the X-ray tube assembly 180 receives a downward force that makes the column 170 tilt forward, the two third rolling bearings abut against the bend edge 1224 at the same time, so that the contact reference of the third rolling bearings with the bend edge 1224 is line contact. Of course, the third rolling bearings may also be replaced with other types of bearing structures or blocky structures as long as they are capable of abutting against the bend edge 1224.

It should be noted that in the implementation where the cylinder 124 is located below the rail seat body 122, the first abutting member 161 is located on the front side of the bend edge 1224 and abuts against the bend edge 1224, and the second abutting member 162 is located on the rear side of the cylinder 124 and abuts against the cylinder 124. In this case, the second auxiliary support member 163 of the medical imaging device 100 is designed to be located on the front side of the cylinder 124 and have a spacing from the cylinder 124 along the Y direction, and the third auxiliary support member 164 is designed to be located on the rear side of the bend edge 1224 and have a spacing from the bend edge 1224 along the Y direction. The spacings are configured such that when the X-ray tube assembly 180 in the working position receives a downward acting force and the column 170 has a tendency to overturn forward, the second auxiliary support member 163 is capable of abutting against the cylinder 124 and the third auxiliary support member 164 is capable of abutting against the bend edge 1224, thereby improving the stability of the column 170.

The first abutting member 161 and the second abutting member 162 may be rolling bearings or blocky structures as long as they are capable of abutting against the sliding seat 120. In a preferred example, the first abutting member 161 and the second abutting member 162 are both rolling bearings, and arc surfaces of the rolling bearings abut against the sliding seat 120. In this way, on the premise of ensuring that the first abutting member 161 and the second abutting member 162 can withstand the support of the sliding seat 120 and have a limiting function, the small friction coefficient of the rolling bearings helps in alleviating the effect of the friction between the first abutting member 161 and second abutting member 162 and the sliding seat 120 on the sliding of the bracket 130.

FIG. 12 is a partial schematic diagram I of the bracket 130 shown in FIG. 6. As shown in FIG. 12, the support plate 131 may be provided with a first through hole. The second abutting member 162 is mounted in the first through hole, and a part of the second abutting member 162 protrudes out of the support plate 131 through the first through hole so as to abut against the cylinder 124 or the bend edge 1224. In this way, on the premise that the second abutting member 162 has a high structural strength to withstand the large supporting force provided by the sliding seat 120, the distance between the support plate 131 and the slideway 121 in the Y direction can be reduced, and the bracket 130 has high stability.

FIG. 13 is a partial schematic diagram II of the bracket 130 shown in FIG. 6. In some embodiments of the disclosure, as shown in FIG. 13, the medical imaging device 100 further includes a fourth auxiliary support member 165. The fourth auxiliary support member 165 is provided with two deep groove ball bearings 1651. The two deep groove ball bearings 1651 are connected to the support plate 131 and spaced part along the X direction. Arc surfaces of the two deep groove ball bearings 1651 both contact the bend edge 1224. In this way, the sliding seat 120 also provides a support for the fourth auxiliary support member 165, so that more positions on the bracket 130 abut against the sliding seat 120, which can improve the force applied on the moving component 140. Thus, the friction between the moving component 140 and the slideway 121 is reduced, which helps in reducing noise generated when the moving component 140 moves along the slideway 121. The deep groove ball bearings 1651 have a small friction coefficient, which helps in reducing noise generated by the friction between the deep groove ball bearings 1651 and the bend edge 1224. Of course, the deep groove ball bearings 1651 may also be replaced with rolling bearings or other structures, which is not limited in this embodiment. The number of the deep groove ball bearings 1651 is two, so that the contact reference of the two deep groove ball bearings 1651 with the bend edge 1224 is line contact, thereby ensuring both of the deep groove ball bearings to be in good contact with the bend edge 1224. Of course, in some embodiments, the number of the deep groove ball bearings 1651 may also be one, three, four or more, which is not limited in this embodiment.

Specifically, the support plate 131 may be provided with a second through hole. The fourth auxiliary support member 165 is further provided with a fixed plate 1652. The fixed plate 1652 is mounted in the second through hole. The two deep groove ball bearings 1651 are respectively connected to two ends of the fixed plate 1652 along the X direction. In this way, a part of the deep groove ball bearing 1651 protrudes out of the support plate 131 through the second through hole so as to abut against the bend edge 1224.

In other embodiments of the disclosure, the fourth auxiliary support member 165 is provided with two deep groove ball bearings 1651. The two deep groove ball bearings 1651 may be connected to the first mounting plate 132, and arc surfaces of the two deep groove ball bearing 1651 both abut against the cylinder 124. Thus, more positions on the bracket 130 abut against the sliding seat 120, which can also improve the force applied on the moving component 140, which helps in reducing noise generated when the moving component 140 moves along the slideway 121.

On the basis of the above embodiments, the sliding seat 120 may include a supporting table 125. The supporting table 125 is fixedly arranged on the bed 110, and the rail seat body 122 and the cylinder 124 may both be fastened and connected to the supporting table 125. By providing the supporting table 125, the supporting table 125 provides a support for the rail seat body 122, the cylinder 124, the bracket 130 and the column 170. The rail seat body 122 and the cylinder 124 are connected to the supporting table 125 by, but not limited to, soldering, a snap fit joint or a screw joint. As shown in FIG. 9, the rail seat body 122 may be connected to a bottom surface of the supporting table 125, and the cylinder 124 is connected to a top surface of the supporting table 125. Alternatively, in other embodiments, the rail seat body 122 may be connected to the top surface of the supporting table 125, and the cylinder 124 is connected to the bottom surface of the supporting table 125.

Schematically, as shown in FIG. 3, the medical imaging device 100 may further include a housing 126. The housing 126 is fixedly connected to the bed 110. A rear side of the housing 126 has an opening. The supporting table 125 is located in the opening and fixedly connected to the housing 126. Thus, the housing 126 covers the sliding seat 120, so that the medical imaging device 100 has a good overall visual effect.

The disclosure has been shown and explained in detail above through the accompanying drawings and preferred embodiments. However, the disclosure is not limited to these disclosed embodiments, and other solutions deduced by those skilled in the art are also possible.

## Claims

1. A medical imaging device (100), wherein the medical imaging device (100) comprises:
a sliding seat (120), fixedly arranged on a bed (110) of the medical imaging device (100), the sliding seat (120) having a slideway (121) extending along a length direction (X) of the bed (110);
a bracket (130), provided with a moving component (140) and a restraining member (160), the moving component (140) moving along the slideway (121), and the restraining member (160) being fitted with the sliding seat (120) to limit a displacement of the bracket (130) in a width direction (Y) of the bed (110);
a column (170), vertically arranged on the bracket (130); and
an X-ray tube assembly (180), arranged on the column (170), wherein
the sliding seat (120) comprises a rail seat body (122), the rail seat body (122) being provided with a bottom edge (1221), a connecting edge (1222), a top edge (1223) and a bend edge (1224) that are connected sequentially, the bottom edge (1221) and the top edge (1223) being arranged opposite to each other, the connecting edge (1222) and the bend edge (1224) being opposite to each other and both perpendicular to the bottom edge (1221), the bottom edge (1221), the connecting edge (1222), the top edge (1223) and the bend edge (1224) together forming the slideway (121), and the moving component (140) being located in the slideway (121) and contacts the bottom edge (1221), **characterized in that**
the medical imaging device (100) further comprises a first auxiliary support member (150), wherein the first auxiliary support member (150) is located in the slideway (121); along a height direction (Z) of the bed (110), a distance between the first auxiliary support member (150) and a bottom surface of the slideway (121) is a first distance, and a distance between the first auxiliary support member (150) and a top surface of the slideway (121) is a second distance; and the second distance is configured such that when the X-ray tube assembly (180) rotates around a central axis of the column (170) to make the column (170) have a tendency to overturn, the first auxiliary support member (150) abuts against the top edge (1223).

2. The medical imaging device (100) according to claim 1, wherein along a height direction (Z) of the bed (110), there is a gap between the moving component (140) and a top surface of the slideway (121).

3. The medical imaging device (100) according to claim 1, wherein the moving component (140) comprises two first support bearings (141), the two first support bearings (141) being spaced apart along the length direction (X) of the bed (110).

4. The medical imaging device (100) according to claim 1, wherein a dodging opening is formed between the bend edge (1224) and the bottom edge (1221), the moving component (140) is connected to the bracket (130) through a connecting member (143), the dodging opening is configured to allow the connecting member (143) to pass through, and a size of the moving component (140) along a height direction (Z) of the bed (110) is greater than a size of the dodging opening along the height direction (Z) of the bed (110).

5. The medical imaging device (100) according to claim 1, wherein the first auxiliary support member (150) comprises two first rolling bearings, the two first rolling bearings being spaced apart along the length direction (X) of the bed (110), and the first rolling bearings being connected to the bracket (130) through a first eccentric shaft (151) such that the second distance is adjustable.

6. The medical imaging device (100) according to any one of claims 1 to 5, wherein the sliding seat (120) further comprises a cylinder (124), the cylinder (124) extending along the length direction (X) of the bed (110); and
the restraining member (160) comprises a pair of first abutting members (161), the first abutting members (161) being located on a front side of the cylinder (124) away from the column (170) and abutting against the cylinder (124), or the first abutting members (161) being located on a front side of the bend edge (1224) away from the column (170) and abutting against the bend edge (1224); and
a pair of second abutting members (162), the second abutting members (162) being located on a rear side of the cylinder (124) near the column (170) and abutting against the cylinder (124), or the second abutting members (162) being located on a rear side of the bend edge (1224) near the column (170) and abutting against the bend edge (1224).

7. The medical imaging device (100) according to claim 6, wherein the cylinder (124) is located above the rail seat body (122), the first abutting members (161) abut against the cylinder (124), and the second abutting members (162) abut against the bend edge (1224);
the medical imaging device (100) further comprises a second auxiliary support member (163), the second auxiliary support member (163) being located on the rear side of the cylinder (124), and along the width direction (Y) of the bed (110), a distance between the second auxiliary support member (163) and the cylinder (124) being a third distance; and
a third auxiliary support member (164), the third auxiliary support member (164) being located on the front side of the bend edge (1224) and in the slideway (121), and along the width direction (Y) of the bed (110), a distance between the third auxiliary support member (164) and the bend edge (1224) being a fourth distance; and
the third distance and the fourth distance are configured such that when the X-ray tube assembly (180) receives an acting force to make the column (170) have a tendency to overturn, the second auxiliary support member (163) is capable of abutting against the cylinder (124) and the third auxiliary support member (164) is capable of abutting against the bend edge (1224).

8. The medical imaging device (100) according to claim 7, wherein
the second auxiliary support member (163) comprises two second rolling bearings, the two second rolling bearings being spaced apart along the length direction (X) of the bed (110), and the second rolling bearings being connected to the bracket (130) through a second eccentric shaft such that the third distance is adjustable; and
the third auxiliary support member (164) comprises two third rolling bearings, the two third rolling bearings being spaced apart along the length direction (X) of the bed (110), and the third rolling bearings being connected to the bracket (130) through a third eccentric shaft such that the fourth distance is adjustable.

9. The medical imaging device (100) according to claim 8, wherein the bracket (130) comprises a support plate (131), and a first mounting plate (132) and a second mounting plate (133) that are perpendicularly connected to the support plate (131), the first abutting members (161) and the second auxiliary support member (163) being fixed to the first mounting plate (132), and the second abutting members (162) and the third auxiliary support member (164) being fixed to the second mounting plate (133).

10. The medical imaging device (100) according to claim 8, further comprising a fourth auxiliary support member (165) fixedly arranged on the bracket (130), wherein the fourth auxiliary support member (165) is located on the rear side of the bend edge (1224) and abuts against the bend edge (1224), or the fourth auxiliary support member (165) is located on the rear side of the cylinder (124) and abuts against the cylinder (124).

11. The medical imaging device (100) according to claim 8, wherein the sliding seat (120) further comprises a supporting table (125) fixedly arranged on the bed (110), the cylinder (124) and the rail seat body (122) being both fastened and connected to the supporting table (125), one of the cylinder (124) and the rail seat body (122) being connected to a top surface of the supporting table (125), and the other being connected to a bottom surface of the supporting table (125)

## Patentansprüche

1. Medizinische Bildgebungsvorrichtung (100), wobei die medizinische Bildgebungsvorrichtung (100) Folgendes umfasst:
einen Schiebesitz (120), der fest an einem Bett (110) der medizinischen Bildgebungsvorrichtung (100) angeordnet ist, wobei der Schiebesitz (120) eine Gleitbahn (121) aufweist, die sich entlang einer Längenrichtung (X) des Betts (110) erstreckt,
eine Halterung (130), die mit einer beweglichen Komponente (140) und einem Rückhalteglied (160) versehen ist, wobei sich die bewegliche Komponente (140) entlang der Gleitbahn (121) bewegt und das Rückhalteglied (160) mit dem Schiebesitz (120) ausgestattet ist, um eine Verschiebung der Halterung (130) in einer Breitenrichtung (Y) des Betts (110) zu begrenzen,
eine vertikal an der Halterung (130) angeordnete Säule (170) und
eine an der Säule (170) angeordnete Röntgenröhrenbaugruppe (180), wobei
der Schiebesitz (120) einen Schienensitzkörper (122) umfasst, wobei der Schienensitzkörper (122) mit einer Unterkante (1221), einer Verbindungskante (1222), einer Oberkante (1223) und einer gebogenen Kante (1224) versehen ist, die aufeinanderfolgend verbunden sind, wobei die Unterkante (1221) und die Oberkante (1223) einander gegenüber angeordnet sind, wobei die Verbindungskante (1222) und die gebogene Kante (1224) einander gegenüberliegen und beide senkrecht zu der Unterkante (1221) verlaufen, wobei die Unterkante (1221), die Verbindungskante (1222), die Oberkante (1223) und die gebogene Kante (1224) zusammen die Gleitbahn (121) bilden und wobei die bewegliche Komponente (140) in der Gleitbahn (121) angeordnet ist und die Unterkante (1221) kontaktiert, **dadurch gekennzeichnet, dass**
die medizinische Bildgebungsvorrichtung (100) ferner ein erstes Hilfsstützglied (150) umfasst, wobei das erste Hilfsstützglied (150) in der Gleitbahn (121) angeordnet ist, wobei entlang einer Höhenrichtung (Z) des Betts (110) ein Abstand zwischen dem ersten Hilfsstützglied (150) und einer unteren Fläche der Gleitbahn (121) ein erster Abstand ist und ein Abstand zwischen dem ersten Hilfsstützglied (150) und einer oberen Fläche der Gleitbahn (121) ein zweiter Abstand ist und der zweite Abstand so ausgestaltet ist, dass das erste Hilfsstützglied (150) an der Oberkante (1223) anliegt, wenn sich die Röntgenröhrenbaugruppe (180) um eine Mittelachse der Säule (170) dreht, so dass die Säule (170) umkippen kann.

2. Medizinische Bildgebungsvorrichtung (100) nach Anspruch 1, wobei sich entlang einer Höhenrichtung (Z) des Betts (110) ein Spalt zwischen der beweglichen Komponente (140) und einer oberen Fläche der Gleitbahn (121) befindet.

3. Medizinische Bildgebungsvorrichtung (100) nach Anspruch 1, wobei die bewegliche Komponente (140) zwei erste Stützlager (141) umfasst, wobei die zwei ersten Stützlager (141) entlang der Längenrichtung (X) des Betts (110) beabstandet sind.

4. Medizinische Bildgebungsvorrichtung (100) nach Anspruch 1, wobei eine Ausweichöffnung zwischen der gebogenen Kante (1224) und der Unterkante (1221) gebildet ist, die bewegliche Komponente (140) durch ein Verbindungsglied (143) mit der Halterung (130) verbunden ist, die Ausweichöffnung so ausgestaltet ist, dass das Verbindungsglied (143) hindurchgehen kann, und eine Größe der beweglichen Komponente (140) entlang einer Höhenrichtung (Z) des Betts (110) größer als eine Größe der Ausweichöffnung entlang der Höhenrichtung (Z) des Betts (110) ist.

5. Medizinische Bildgebungsvorrichtung (100) nach Anspruch 1, wobei das erste Hilfsstützglied (150) zwei erste Wälzlager umfasst, wobei die zwei ersten Wälzlager entlang der Längenrichtung (X) des Betts (110) beabstandet sind und die ersten Wälzlager durch eine erste Exzenterwelle (151) mit der Halterung (130) verbunden sind, so dass der zweite Abstand verstellbar ist.

6. Medizinische Bildgebungsvorrichtung (100) nach einem der Ansprüche 1 bis 5, wobei der Schiebesitz (120) ferner einen Zylinder (124) umfasst, wobei sich der Zylinder (124) entlang der Längenrichtung (X) des Betts (110) erstreckt, und
das Rückhalteglied (160) ein Paar erste Anlageglieder (161) umfasst, wobei die ersten Anlageglieder (161) an einer Vorderseite des Zylinders (124) entfernt von der Säule (170) angeordnet sind und an dem Zylinder (124) anliegen, oder wobei die ersten Anlageglieder (161) an einer Vorderseite der gebogenen Kante (1224) entfernt von der Säule (170) angeordnet sind und an der gebogenen Kante (1224) anliegen, sowie
ein Paar zweite Anlageglieder (162), wobei die zweiten Anlageglieder (162) an einer Rückseite des Zylinders (124) nahe der Säule (170) angeordnet sind und an dem Zylinder (124) anliegen, oder wobei die zweiten Anlageglieder (162) an einer Rückseite der gebogenen Kante (1224) nahe der Säule (170) angeordnet sind und an der gebogenen Kante (1224) anliegen.

7. Medizinische Bildgebungsvorrichtung (100) nach Anspruch 6, wobei der Zylinder (124) über dem Schienensitzkörper (122) angeordnet ist, die ersten Anlageglieder (161) an dem Zylinder (124) anliegen und die zweiten Anlageglieder (162) an der gebogenen Kante (1224) anliegen,
wobei die medizinische Bildgebungsvorrichtung (100) ferner ein zweites Hilfsstützglied (163) umfasst, wobei das zweite Hilfsstützglied (163) an der Rückseite des Zylinders (124) und entlang der Breitenrichtung (Y) des Betts (110) angeordnet ist, wobei ein Abstand zwischen dem zweiten Hilfsstützglied (163) und dem Zylinder (124) ein dritter Abstand ist, sowie
ein drittes Hilfsstützglied (164), wobei das dritte Hilfsstützglied (164) an der Vorderseite der gebogenen Kante (1224) und in der Gleitbahn (121) und entlang der Breitenrichtung (Y) des Betts (110) angeordnet ist, wobei ein Abstand zwischen dem dritten Hilfsstützglied (164) und der gebogenen Kante (1224) ein vierter Abstand ist, und
der dritte Abstand und der vierte Abstand so ausgestaltet sind, dass das zweite Hilfsstützglied (163) in der Lage ist, an dem Zylinder (124) anzuliegen, und das dritte Hilfsstützglied (164) in der Lage ist, an der gebogenen Kante (1224) anzuliegen, wenn eine Kraft auf die Röntgenröhrenbaugruppe (180) einwirkt, die die Säule (170) zu einem Umkippen bringen kann.

8. Medizinische Bildgebungsvorrichtung (100) nach Anspruch 7, wobei
das zweite Hilfsstützglied (163) zwei zweite Wälzlager umfasst, wobei die zwei zweiten Wälzlager entlang der Längenrichtung (X) des Betts (110) beabstandet sind und die zweiten Wälzlager über eine zweite Exzenterwelle mit der Halterung (130) verbunden sind, so dass der dritte Abstand verstellbar ist, und
das dritte Hilfsstützglied (164) zwei dritte Wälzlager umfasst, wobei die zwei dritten Wälzlager entlang der Längenrichtung (X) des Betts (110) beabstandet sind und die dritten Wälzlager über eine dritte Exzenterwelle mit der Halterung (130) verbunden sind, so dass der vierte Abstand verstellbar ist.

9. Medizinische Bildgebungsvorrichtung (100) nach Anspruch 8, wobei die Halterung (130) eine Stützplatte (131) und eine erste Montageplatte (132) und eine zweite Montageplatte (133) umfasst, die senkrecht mit der Stützplatte (131) verbunden sind, wobei die ersten Anlageglieder (161) und das zweite Hilfsstützglied (163) an der ersten Montageplatte (132) befestigt sind und die zweiten Anlageglieder (162) und das dritte Hilfsstützglied (164) an der zweiten Montageplatte (133) befestigt sind.

10. Medizinische Bildgebungsvorrichtung (100) nach Anspruch 8, ferner umfassend ein viertes Hilfsstützglied (165), das fest an der Halterung (130) angeordnet ist, wobei das vierte Hilfsstützglied (165) an der Rückseite der gebogenen Kante (1224) angeordnet ist und an der gebogenen Kante (1224) anliegt, oder das vierte Hilfsstützglied (165) an der Rückseite des Zylinders (124) angeordnet ist und an dem Zylinder (124) anliegt.

11. Medizinische Bildgebungsvorrichtung (100) nach Anspruch 8, wobei der Schiebesitz (120) ferner einen Stütztisch (125) umfasst, der fest auf dem Bett (110) angeordnet ist, wobei sowohl der Zylinder (124) als auch der Schienensitzkörper (122) an dem Stütztisch (125) befestigt und mit diesem verbunden sind, wobei entweder der Zylinder (124) oder der Schienensitzkörper (122) mit einer oberen Fläche des Stütztischs (125) verbunden ist und der jeweils andere mit einer Bodenfläche des Stütztischs (125) verbunden ist.

## Revendications

1. Dispositif d'imagerie médicale (100), le dispositif d'imagerie médicale (100) comprenant :
un siège coulissant (120), agencé de manière fixe sur un lit (110) du dispositif d'imagerie médicale (100), le siège coulissant (120) ayant une glissière (121) s'étendant le long d'une direction de longueur (X) du lit (110) ;
un support (130), pourvu d'un composant mobile (140) et d'un élément de retenue (160), le composant mobile (140) se déplaçant le long de la glissière (121), et l'élément de retenue (160) étant ajusté avec le siège coulissant (120) pour limiter un déplacement du support (130) dans une direction de largeur (Y) du lit (110) ;
une colonne (170), agencée verticalement sur le support (130) ; et
un ensemble tube à rayons X (180), agencé sur la colonne (170),
le siège coulissant (120) comprenant un corps de siège à rail (122), le corps de siège à rail (122) étant pourvu d'un bord inférieur (1221), d'un bord de connexion (1222), d'un bord supérieur (1223) et d'un bord courbé (1224) qui sont connectés séquentiellement, le bord inférieur (1221) et le bord supérieur (1223) étant agencés l'un en face de l'autre, le bord de connexion (1222) et le bord courbé (1224) étant opposés l'un à l'autre et tous deux perpendiculaires au bord inférieur (1221), le bord inférieur (1221), le bord de connexion (1222), le bord supérieur (1223) et le bord courbé (1224) formant ensemble la glissière (121), et le composant mobile (140) étant situé dans la glissière (121) et entrant en contact avec le bord inférieur (1221), **caractérisé en ce que**
le dispositif d'imagerie médicale (100) comprend en outre un premier élément de support auxiliaire (150), le premier élément de support auxiliaire (150) étant situé dans la glissière (121) ; le long d'une direction de hauteur (Z) du lit (110), une distance entre le premier élément de support auxiliaire (150) et une surface inférieure de la glissière (121) étant une première distance, et une distance entre le premier élément de support auxiliaire (150) et une surface supérieure de la glissière (121) étant une deuxième distance ; et la deuxième distance étant configurée de telle sorte que lorsque l'ensemble tube à rayons X (180) tourne autour d'un axe central de la colonne (170) pour donner à la colonne (170) une tendance à basculer, le premier élément de support auxiliaire (150) vient en butée contre le bord supérieur (1223).

2. Dispositif d'imagerie médicale (100) selon la revendication 1, le long d'une direction de hauteur (Z) du lit (110), un écart existant entre le composant mobile (140) et une surface supérieure de la glissière (121).

3. Dispositif d'imagerie médicale (100) selon la revendication 1, le composant mobile (140) comprenant deux premiers paliers de support (141), les deux premiers paliers de support (141) étant espacés le long de la direction de longueur (X) du lit (110).

4. Dispositif d'imagerie médicale (100) selon la revendication 1, une ouverture d'évitement étant formée entre le bord courbé (1224) et le bord inférieur (1221), le composant mobile (140) étant connecté au support (130) par l'intermédiaire d'un élément de connexion (143), l'ouverture d'évitement étant configurée pour permettre à l'élément de connexion (143) de passer à travers, et une taille du composant mobile (140) le long d'une direction de hauteur (Z) du lit (110) étant supérieure à une taille de l'ouverture d'évitement le long de la direction de hauteur (Z) du lit (110).

5. Dispositif d'imagerie médicale (100) selon la revendication 1, le premier élément de support auxiliaire (150) comprenant deux premiers paliers à roulement, les deux premiers paliers à roulement étant espacés le long de la direction de longueur (X) du lit (110), et les premiers paliers à roulement étant connectés au support (130) par l'intermédiaire d'un premier arbre excentrique (151) de telle sorte que la deuxième distance soit ajustable.

6. Dispositif d'imagerie médicale (100) selon l'une quelconque des revendications 1 à 5, le siège coulissant (120) comprenant en outre un cylindre (124), le cylindre (124) s'étendant le long de la direction de longueur (X) du lit (110) ; et
l'élément de retenue (160) comprenant une paire de premiers éléments de butée (161), les premiers éléments de butée (161) étant situés sur un côté avant du cylindre (124) éloigné de la colonne (170) et en butée contre le cylindre (124), ou les premiers éléments de butée (161) étant situés sur un côté avant du bord courbé (1224) éloigné de la colonne (170) et en butée contre le bord courbé (1224) ; et
une paire de seconds éléments de butée (162), les seconds éléments de butée (162) étant situés sur un côté arrière du cylindre (124) proche de la colonne (170) et en butée contre le cylindre (124), ou les seconds éléments de butée (162) étant situés sur un côté arrière du bord courbé (1224) proche de la colonne (170) et en butée contre le bord courbé (1224).

7. Dispositif d'imagerie médicale (100) selon la revendication 6, le cylindre (124) étant situé au-dessus du corps de siège à rail (122), les premiers éléments de butée (161) étant en butée contre le cylindre (124), et les seconds éléments de butée (162) étant en butée contre le bord courbé (1224) ;
le dispositif d'imagerie médicale (100) comprenant en outre un deuxième élément de support auxiliaire (163), le deuxième élément de support auxiliaire (163) étant situé sur le côté arrière du cylindre (124), et le long de la direction de largeur (Y) du lit (110), une distance entre le deuxième élément de support auxiliaire (163) et le cylindre (124) étant une troisième distance ; et
un troisième élément de support auxiliaire (164), le troisième élément de support auxiliaire (164) étant situé sur le côté avant du bord courbé (1224) et dans la glissière (121), et le long de la direction de largeur (Y) du lit (110), une distance entre le troisième élément de support auxiliaire (164) et le bord courbé (1224) étant une quatrième distance ; et
la troisième distance et la quatrième distance étant configurées de telle sorte que lorsque l'ensemble tube à rayons X (180) reçoit une force d'action pour donner à la colonne (170) une tendance à basculer, le deuxième élément de support auxiliaire (163) est capable de venir en butée contre le cylindre (124) et le troisième élément de support auxiliaire (164) est capable de venir en butée contre le bord courbé (1224).

8. Dispositif d'imagerie médicale (100) selon la revendication 7,
le deuxième élément de support auxiliaire (163) comprenant deux deuxièmes paliers à roulement, les deux deuxièmes paliers à roulement étant espacés le long de la direction de longueur (X) du lit (110), et les deuxièmes paliers à roulement étant connectés au support (130) par l'intermédiaire d'un deuxième arbre excentrique de telle sorte que la troisième distance soit ajustable ; et
le troisième élément de support auxiliaire (164) comprenant deux troisièmes paliers à roulement, les deux troisièmes paliers à roulement étant espacés le long de la direction de longueur (X) du lit (110), et les troisièmes paliers à roulement étant connectés au support (130) par l'intermédiaire d'un troisième arbre excentrique de telle sorte que la quatrième distance soit ajustable.

9. Dispositif d'imagerie médicale (100) selon la revendication 8, le support (130) comprenant une plaque de support (131), et une première plaque de montage (132) et une seconde plaque de montage (133) qui sont connectées perpendiculairement à la plaque de support (131), les premiers éléments de butée (161) et le deuxième élément de support auxiliaire (163) étant fixés à la première plaque de montage (132), et les seconds éléments de butée (162) et le troisième élément de support auxiliaire (164) étant fixés à la seconde plaque de montage (133).

10. Dispositif d'imagerie médicale (100) selon la revendication 8, comprenant en outre un quatrième élément de support auxiliaire (165) agencé de manière fixe sur le support (130), le quatrième élément de support auxiliaire (165) étant situé sur le côté arrière du bord courbé (1224) et en butée contre le bord courbé (1224), ou le quatrième élément de support auxiliaire (165) étant situé sur le côté arrière du cylindre (124) et en butée contre le cylindre (124).

11. Dispositif d'imagerie médicale (100) selon la revendication 8, le siège coulissant (120) comprenant en outre une table de support (125) agencée de manière fixe sur le lit (110), le cylindre (124) et le corps de siège à rail (122) étant tous deux fixés et connectés à la table de support (125), l'un du cylindre (124) et du corps de siège à rail (122) étant connecté à une surface supérieure de la table de support (125), et l'autre étant connecté à une surface inférieure de la table de support (125).
